# FASCICULE DE BREVET EUROPEEN

(11) **EP 0 710 658 B1**
(45) Date de publication et mention de la délivrance du brevet: **04.10.2001**
(21) Numéro de dépôt: 95402437.8
(22) Date de dépôt: 02.11.1995
(51) Int. Cl.: C07D 263/28, C07D 413/12, A61K 31/42

(54) **Nouvelles 5-(aryloxymethyl)oxazolines, leur procédé de préparation et les compositions pharmaceutiques qui les contiennent**
5-(Aryloxymethyl)oxazoline, Verfahren zu ihrer Herstellung und pharmazeutische Zusammensetzungen die sie enthalten
5-(Aryloxymethyl)oxazolines, process for their preparation and pharmaceutical compositions containing them

(30) Priorité: 03.11.1994 FR 9413087
(43) Date de publication de la demande: 08.05.1996
(73) Titulaire: ADIR ET COMPAGNIE, 92415 Courbevoie Cédex (FR)
(72) Inventeur: Jarry, Christian, F-33370 Artigues-pres-Bordeaux (FR); Forfar, Isabelle, F-33000 Bordeaux (FR); Bosc, Jean-Jacques, F-33370 Pompignac (FR); Renard, Pierre, F-78000 Versailles (FR); Scalbert, Elizabeth, F-92100 Boulogne (FR); Guardiola, Béatrice, F-92210 Saint Cloud (FR)

(56) Documents cités:
- EP-A- 0 392 929
- US-A- 3 637 726
- US-A- 3 818 028
- BULLETIN DE LA SOCIETE DE PHARMACIE DE BORDEAUX, vol. 120, pages 153-162, CHRISTIAN JARRY ET AL 'Synthése et étude structurale d'amino-2-oxazolines-2 à action antihypertensive'

## Description

L'invention concerne de nouvelles 5-(aryloxyméthyl)oxazolines, leur procédé de préparation et les compositions pharmaceutiques qui les contiennent.

On connaît dans la littérature des 5-[(2,4,6-trihalogénophénoxy)méthyl]-2-oxazolines (brevet US-A-3,818,028) décrits comme anti-depresseurs et des 5-[(3,4-dihalogénophénoxy)méthyl]-2-oxazolines (brevet US-A-3,637,726) décrites comme agents anti-microbiens.

La publication par C. Jarry et coll. (*Eur. J. Med. Chem., 25*(4), (1990), 379-382) présente des phenoxyméthyloxazolines utilisées comme produits de départ dans la synthèse de phénacylimino-oxazolidines.

Des 5-(phénylphénoxyméthyl)-2-oxazolines douées de propriétés anti-dépressives ont également été décrites dans le brevet EP-A-392929.

Enfin, C. Jarry et coll. (*Bull. Soc. Pharm. Bordeaux,* (1981), *120,* 153-162) ont démontré les propriétés anti-hypertensives de certaines 2-amino-2-oxazolines. Or, ces composés possèdent de plus une activité excitante d'origine centrale.

Le but de la présente invention consiste à trouver de nouveaux composés doués de propriétés anti-hypertensives et dépourvus des effets secondaires d'origine centrale.

Ainsi, la présente invention concerne de nouvelles 5-(aryloxyméthyl)oxazolines possédant une puissante affinité pour les récepteurs de l'imidazoline I₁ (récepteur I₁).

De façon surprenante, cette affinité est sélective puisque les composés de l'invention ne possédent pas d'affinité comparable pour les récepteurs de l'imidazoline I₂ (récepteurs I₂) ainsi que pour les récepteurs adrénergiques α₁ et α₂, responsables des effets secondaires d'origine centrale.

Par conséquent, les composés de l'invention seront avantageusement utilisés en thérapeutique dans le traitement des pathologies liées aux récepteurs I₁, en particulier dans l'hypertension.

L'absence d'affinité pour les récepteurs α permet d'éviter les effets indésirables neuropharmacologiques centraux habituellement rencontrés dans ce type de traitement.

L'invention concerne les composés de formule (I) : 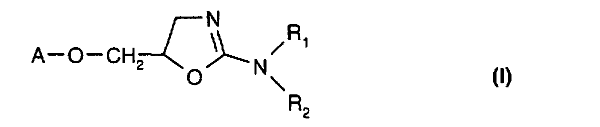 dans laquelle :
- R₁ et R₂ sont choisis indépendamment l'un de l'autre parmi l'hydrogène ou un radical alkyle,
- A est choisi parmi les radicaux : 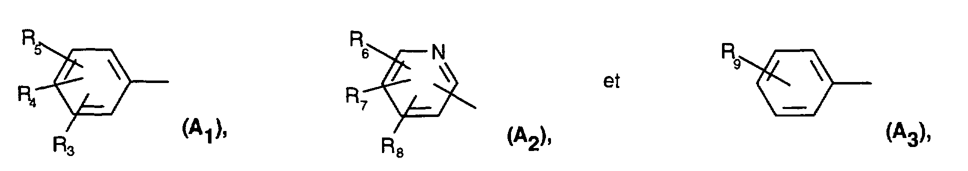 dans lesquels :

- R₃ représente un radical choisi parmi halogène, alkyle, alkoxy, amino, alkylamino, dialkylamino, carboxy, alkylcarbonyle, alkoxycarbonyle, trifluorométhyle, et cyano,
- R₄, R₅, R₆, R₇ et R₈ sont choisis chaucun indépendamment l'un de l'autre parmi l'hydrogène, un halogène, un radical alkyle, alkoxy, amino, alkylamino, dialkylamino, carboxy, alkoxycarbonyle, cyano et trifluorométhyle,
- R₉ représente le radical nitro,
avec la réserve que A ne peut pas représenter un groupement choisi parmi : dihalogénophényle, trihalogénophényle, 4-méthylphényle, 4-chlorophényle, 4-méthoxyphényle, 4-tert-butylphényle et 3-(diméthylamino)phényle, leurs isomères optiques sous forme pure ou en mélange, ainsi que leurs sels d'addition à un acide ou une base, pharmaceutiquement acceptables,
étant entendu que : les termes "alkyle" et "alkoxy" représentent des radicaux linéaires ou ramifiés contenant de 1 à 6 atomes de carbone.

L'invention concerne particulièrement les composés de formule (I) dans laquelle A représente le radical de formule (A₁) tel que défini dans la formule (I).

L'invention concerne particulièrement les composés de formule (I) dans laquelle A représente le radical de formule (A₂) tel que défini dans la formule (I).

L'invention concerne particulièrement les composés de formule (I) dans laquelle A représente le radical de formule (A₄) : 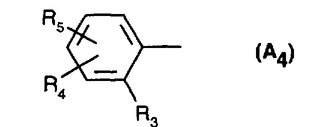 dans laquelle R₃, R₄ et R₅ sont tels que définis dans la formule (I).

L'invention concerne plus particulièrement les composés de formule (I) dans laquelle A représente le radical de formule (A₆) : 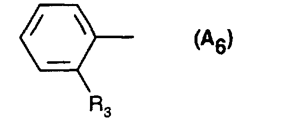 cas particulier du radical (A₁) tel que défini dans la formule (I) dans lequel R₄ et R₅ représentent chacun l'hydrogène.

L'invention concerne par exemple les composés de formule (I) dans lesquels A représente le radical de formule A₆ et R₃ est choisi parmi halogène, alkyle et alkoxy, en particulier alkyle.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

Plus particulièrement, les radicaux alkyles présents dans la formule (I) sont choisis parmi méthyle, éthyle, n-propyle, isopropyle, n-butyle, isobutyle, sec-butyle, tert-butyle, pentyle, et hexyle.

Les radicaux alkoxy présents dans la formule (I) sont choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) sont choisis parmi le brome, le chlore, le fluor, et l'iode.

L'invention concerne également le procédé de préparation des composés de formule (I), caractérisé en ce qu'on fait réagir un composé de formule (II) : 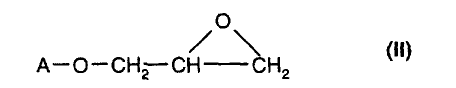 dans laquelle A est tel que défini dans la formule (I) avec le cyanamide de sodium afin d'obtenir un composé de formule (I/a) : 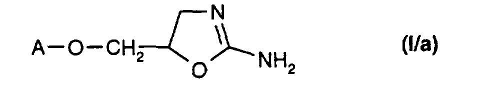 dans laquelle A est tel que défini précédemment, composé de formule (I/a) qui est ensuite mono- ou di-alkylé sur l'amine primaire afin d'obtenir le composé de formule (I/b) correspondant au composé de formule (I) dans lesquels R₁ et/ou R₂ représentent un alkyle, les composés de formule (I/a) et (I/b) formant l'ensemble des composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels isomères optiques, par des techniques classiques de séparation,
- ou transformés par un acide ou une base, en sels pharmaceutiquement acceptables.

Les matières premières utilisées dans le procédé de préparation des composés de formule (I) sont soit commerciales soit aisément accessibles à l'homme du métier.

Par exemple, les composés de formule (II) sont obtenus de manière classique à partir du phénol ou du pyridinol correspondant et de l'épichlorhydrine.

Pour obtenir le cyanamide de sodium, on fait réagir l'éthylate de sodium sur le cyanamide dans l'éthanol.

Les composés de formule (I) possèdent des propriétés pharmacologiques très intéressantes pour le clinicien et le médecin.

Les composés de l'invention et les compositions pharmaceutiques les contenant s'avèrent être utiles pour le traitement des troubles liés aux récepteurs I₁.

Il est en effet connu que les récepteurs I₁ sont les médiateurs d'une action hypotensive de type centrale, telle qu'elle a été montrée dans les études sur les effets de la rilménidine.

Il est également connu que les récepteurs I₁ interviennent dans la stimulation de la libération d'insuline par les cellules β du pancréas (Schulz et al., *Naunyn-Schmiedeberg's Arch. Pharmacol*., (1989), *340* (6), 712-714).

Les récepteurs I₁ sont également impliqués dans l'anémie, notamment l'anémie à hématies falciformes, et la prolifération cancéreuse.

Or, l'étude pharmacologique des dérivés de l'invention a montré qu'ils n'étaient pas toxiques, doués d'une très haute affinité sélective pour les récepteurs I₁.

Ceci permet d'établir que les produits de l'invention sont utiles dans le traitement des pathologies cardiovasculaires et notamment de l'hypertension en particulier de l'hypertension artérielle essentielle, ainsi que dans le traitement du diabète, de l'anémie, notamment l'anémie à hématies falciformes et du cancer.

Les composés seront utilisés de préférence dans les traitements de l'hypertension artérielle essentielle.

La présente invention a également pour objet les compositions pharmaceutiques contenant les produits de formule (I) ou le cas échéant un de leurs sels d'addition à un acide ou une base, pharmaceutiquement acceptables en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per- ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 100 mg par 24 heures en 1 ou 2 prises, plus particulièrement entre 1 et 10 mg, par exemple entre 1 et 2 mg.

Les exemples suivants illustrent l'invention, mais ne la limitent en aucune façon.

### EXEMPLES 1 A 25 :

En procédant comme il est décrit dans le mode opératoire général et en utilisant l'époxyde convenablement substitué, on obtient les composés des exemples suivants :

### Mode opératoire général :

La création de l'hétérocycle oxazoline procède par ouverture du cycle époxydique sous l'action du cyanamide de sodium. Cette réaction est réalisée dans le méthanol, ce qui offre l'avantage de rendre le milieu homogène et surtout de prévenir l'hydrolyse du cyanamide de sodium. Les rendements sont satisfaisants.

Dans un réacteur, on introduit 0,2 mole de cyanamide de sodium et 200 cm³ de méthanol anhydre. Quand la dissolution est complète, on ajoute goutte à goutte 0,2 mole d'époxyde ; il convient de maintenir la température au-dessous de + 20°C. Après douze heures d'agitation, la 4,5-dihydro-oxazol-2-ylamine se sépare parfois à l'état solide. On la purifie par cristallisation fractionnée. Lorsque le milieu réactionnel demeure homogène, on évapore le méthanol. Le résidu solide est un mélange de 4,5-dihydro-oxazol-2-ylamine et de méthylate de sodium. Après élimination de ce dernier par lavage à l'eau, on est ramené au cas précédent.

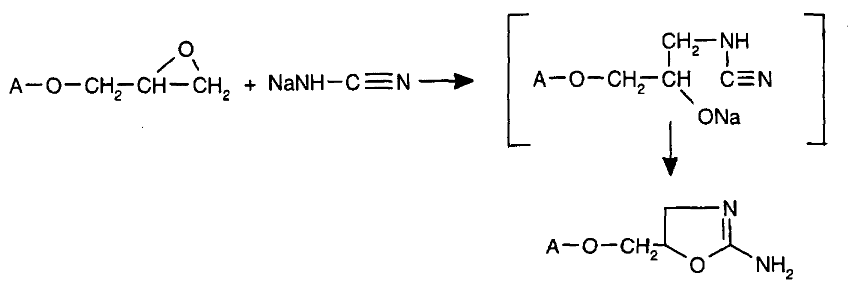

### EXEMPLE 1 : 5-[(2-METHYLPHENOXY)METHYL]-4,5-DIHYDRO-OXAZOL-2-YLAMINE

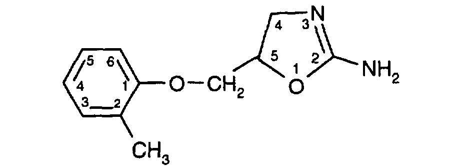

poudre blanche cristalline
Point de fusion (F) = 130°C (heptane) pKa = 8,54 log P_{o/w} = 1,69
moment dipolaire : benzène : 1,80 D dioxanne : 2,07 D
**IR (cm**^{**-1**}**) pastille KBr :** v (NH) : 3410 ; v (C≡N) : 1680
**RMN**^{**1**}**H (CDCl**_{**3**}**) référence interne TMS, δ (ppm) :** 7,0 (m, 4H, arom) ; 4,91 (m, 1H, CH-O) ; 4,88 (s, 2H, NH₂) ; 3,86 (m, 4H, CH₂) ; 2,3 (s, 3H, CH₃)

| **Analyse élémentaire :** | | C₁₁H₁₄N₂O₂ | (M=206) |
|---|---|---|---|
| | % C | %H | %N |
| Calculé | 64,08 | 6,80 | 13,59 |
| Trouvé | 64,09 | 6,84 | 13,52 |
| **Préparation** | Epoxyde : Point d'ébullition (Eb.) : 88°C. (53,31 Pa) | | |
| | Rendement : 49 % | | |
| | Rendement (Oxazoline) : 51 % | | |

### EXEMPLE 2 : 5-[(3-METHYLPHENOXY)METHYL]-4,5-DIHYDRO-OXAZOL-2-YLAMINE

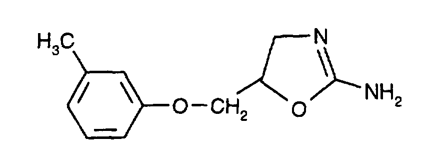

poudre blanche cristalline
F = 130° C (C₂HCl₃)
**IR (cm**^{**-1**}**) pastille KBr** : v (NH) : 3420 ; v (C≡N) : 1685
**RMN**^{**1**}**H (CDCl**_{**3**}**), référence interne TMS,** δ **(ppm) :** 7,00 (m, 4H, arom) ; 4,90 (m, 1H, CH-O) ; 4,30 (s, 2H, NH₂) ; 3,82 (m, 4H, CH₂) ; 2,34 (s, 3H, CH₃)

| **Analyse élémentaire** | | C₁₁H₁₄N₂O₂ | (M=206) |
|---|---|---|---|
| | %C | %H | %N |
| Calculé | 64,08 | 6,80 | 13,59 |
| Trouvé | 64,07 | 6,77 | 13,44 |
| **Préparation** | Epoxyde : Eb. : 100°C. (66,64 Pa) | | |
| | Rendement : 72% | | |
| | Rendement (Oxazoline) : 43 % | | |

### EXEMPLE 3 : 5-[(2-CHLOROPHENOXY)METHYL]-4,5-DIHYDRO-OXAZOL-2-YLAMINE

C₁₀H₁₁N₂O₂Cl (M=226,45)
F = 142° C (C₂HCl₃)
Rendement : 21 %

### EXEMPLE 4 : 5-[(3-CHLOROPHENOXY)METHYL]-4,5-DIHYDRO-OXAZOL-2-YLAMINE

C₁₀H₁₁N₂O₂Cl (M=226,45)
F = 128°C (CCl₄)
Rendement : 24 %

### EXEMPLE 5 : 5-[(2-METHOXYPHENOXY)METHYL]-4,5-DIHYDRO-OXAZOL-2-YLAMINE

C₁₁H₁₄N₂O₃ (M=222)
F = 110°C (C₂HCl₃)
Rendement : 13 %

### EXEMPLE 6 : 5-[(3-METHOXYPHENOXY)METHYL]-4,5-DIHYDRO-OXAZOL-2-YLAMINE

C₁₁H₁₄N₂O₃ (M=222)
F = 110°C
Rendement : 28 %

### EXEMPLE 7: 5-[(2-ETHOXYPHENOXY)METHYL]-4,5-DIHYDRO-OXAZOL-2-YLAMINE

C₁₂H₁₆N₂O₃ (M=236)
F = 130°C (CCl4)
Rendement : 21 %

### EXEMPLE 8 : 5-[(4-ETHYLPHENOXY)METHYL]-4,5-DIHYDRO-OXAZOL-2-YLAMINE

C₁₂H₁₆N₂O₂ (M=220)
F = 172°C (C₂H₅OH)
Rendement : 45 %

### EXEMPLE 9 :5-[(2,6-DIMETHYLPHENOXY)METHYL]-4,5-DIHYDRO-OXAZOL-2-YLAMINE

C₁₂H₁₆N₂O₂ (M=220)
F = 114°C (heptane)
Rendement : 24 %

### EXEMPLE 10 : 5-[(2,4-DIMETHYLPHENOXY)METHYL]-4,5-DIHYDRO-OXAZOL-2-YLAMINE

C₁₂H₁₆N₂O₂ (M=220)
F = 116°C (heptane)
Rendement : 31 %

### EXEMPLE 11 : 5-[(3,4-DIMETHYLPHENOXY)METHYL]-4,5-DIHYDRO-OXAZOL-2-YLAMINE

C₁₂H₁₆N₂O₂ (M=220)
F = 144°C (C₂HCl₃)
Rendement : 21 %

### EXEMPLE 12 : 5-{[4-(1,1-DIMETHYLPROPYL)PHENOXY]METHYL}-4,5-DIHYDROOXAZOL-2-YLAMINE

C₁₅H₂₂N₂O₂ (M=262)
F = 104°C (heptane)
Rendement : 11 %

### EXEMPLE 13 : 5-[(2,3-DIMETHYLPHENOXY)METHYL]-4,5-DIHYDRO-OXAZOL-2-YLAMINE

F = 175°C (C₂H₅OH)

### EXEMPLE 14 : 5-[(3-NITROPHENOXY)METHYL]-4,5-DIHYDRO-OXAZOL-2-YLAMINE

### EXEMPLE 15 : 5-[(4-NITROPHENOXY)METHYL]-4,5-DIHYDRO-OXAZOL-2-YLAMINE

### EXEMPLE 16 : 5-[(2,5-DIMETHYLPHENOXY)METHYL]-4,5-DIHYDRO-OXAZOL-2-YLAMINE

F = 131°C (C₂HCl₃)

### EXEMPLE 17 : 5-[(4-CHLORO-2-METHYLPHENOXY)METHYL]-4,5-DIHYDRO-OXAZOL-2-YLAMINE

F = 130°C (C₂Cl₄)

### EXEMPLE 18 : 5-[(2-ETHYLPHENOXY)METHYL]-4,5-DIHYDRO-OXAZOL-2-YLAMINE

F = 109°C (C₂Cl₄)

### EXEMPLE 19 : 5-[(2-ISOPROPYLPHENOXY)METHYL]-4,5-DIHYDRO-OXAZOL-2-YLAMINE

F = 134°C (C₂Cl₄)

### EXEMPLE 20 : 5-[(2-FLUOROPHENOXY)METHYL]-4,5-DIHYDRO-OXAZOL-2-YLAMINE

F = 124°C (C₂HCl₃)

### EXEMPLE 21 : 5-[(2-NITROPHENOXY)METHYL]-4,5-DIHYDRO-OXAZOL-2-YLAMINE

F = 166°C (toluène)

### EXEMPLE 22 : 5-[(2-CYANOPHENOXY)METHYL]-4,5-DIHYDRO-OXAZOL-2-YLAMINE

F = 146°C (toluène)

### EXEMPLE 23 : 5-[(2-ACETYLPHENOXY)METHYL]-4,5-DIHYDRO-OXAZOL-2-YLAMINE

F = 142°C (C₂Cl₄)

### EXEMPLE 24: 5-[(3-TRIFLUOROMETHYLPHENOXY)METHYL]-4,5-DIHYDRO-OXAZOL-2-YLAMINE

F = 10°C (heptane)

### EXEMPLE 25 : 5-[(2,6-DIMETHOXYPHENOXY)METHYL]-4,5-DIHYDRO-OXAZOL-2-YLAMINE

F = 180°C (C₂HCl₃)

### EXEMPLE 26 : N-n-PROPYL-N-[5-(2-METHYLPHENOXY)METHYL-4,5-DIHYDROOXAZOL-2-YL]AMINE

Dans un réacteur sont introduits 0,01 moles de 5-[(2-méthylphénoxy)méthyl]-4,5-dihydrooxazol-2-ylamine et 100 ml d'acétone. Quand la dissolution est complète, 0,005 moles de 1-bromopropyle sont ajoutés et le milieu est porté à ébullition pendant 48 heures. Après filtration du solide formé et évaporation du solvant, la N-n-propyl-N-[5-(2-méthylphénoxy)méthyl-4,5-dihydro-oxazol-2-yl]amine est séparée par chromatographie sur colonne de silice (éluant chloroforme/ammoniac, 95:5).
C₁₄H₂₀N₂O₂ M = 248
poudre blanche cristalline, F = 108°C (heptane)

### EXEMPLE 27 : N-ETHYL-N-[5-(2-METHYLPHENOXY)METHYL-4,5-DIHYDRO-OXAZOL-2-YL]AMINE

En procédant de manière analogue à celle décrite pour l'exemple 26, mais en remplaçant le 1-bromopropyle par le 1-bromoéthyle, le composé du titre est obtenu.
F = 126°C (heptane)

### EXEMPLE 28 : 5-[(2-METHYLPHENOXY)METHYL]-4,5-DIHYDRO-OXAZOL-2-YLAMINE (Isomère 1 du composé de l'exemple 1).

Cet isomère a été obtenu à partir du composé de l'exemple 1 (racémique) par séparation chromatographique dans les conditions suivantes :
Colonne : CHIRALCEL OD
Eluant : n-heptane/éthanol/diéthylamine, 900:100:0,4
Débit : 1 ml/min.
Injection : 4 µl d'une solution de 4 mg/ml (mélange dichlorométhane/isopropanol, 50:50)
Détection : 220 nm.

### EXEMPLE 29 : 5-[(2-METHYLPHENOXY)METHYL]-4,5-DIHYDRO-OXAZOL-2-YLAMINE (Isomère 2 du composé de l'exemple 1)

Enantiomère du composé décrit à l'exemple 28 obtenu selon le même procédé, à partir du composé de l'exemple 1 (racémique).

### ETUDE PHARMACOLOGIQUE

### EXEMPLE A: PROFIL DE LIAISON AUX RECEPTEURS IMIDAZOLINES I1 ET I2. (ETUDE IN VITRO)

### OBJECTIF :

Mesurer, in vitro, l'affinité de liaison des composés de l'invention aux récepteurs I1 et I2, en déterminant la capacité de ces composés à déplacer les radioligands spécifiques des récepteurs imidazolines I₁ et I₂.

### PROTOCOLE :

Le tableau suivant indique le radioligand utilisé pour marquer le récepteur, le produit et la concentration retenue pour déterminer la fraction non spécifique et le tissu choisi.

| Récepteur ou site | Radioligand | non spécifique | Structure |
|---|---|---|---|
| I1 | [3H]-Clonidine + 10 µM de Norépinéphrine | 10⁻⁵M Clonidine froide | Noyau reticulé latéral de boeuf |
| I2 | [3H]-Idazoxan + 10 µM de Norépinéphrine | 10⁻⁵M Idazoxan | cortex rénal de lapin |

### RESULTATS :

Les résultats obtenus, in vitro sur les récepteurs centraux ou périphériques et avec nos conditions expérimentales, montrent que, les composés de l'invention présentent une très haute affinité pour les sites I₁ (voir tableau 1), alors qu'ils se lient avec une faible affinité aux récepteurs I₂ du cortex rénal de lapin.

**- Tableau 1 -**

| Profil de liaison aux récepteurs I₁ et I₂ | | |
|---|---|---|
| ***EXEMPLE*** | ***K***_{***i***}***(M)*** | |
| | I₁ | I₂ |
| 1 | 8,5.10⁻⁹ | 9,2.10⁻⁷ |
| 9 | 5,7.10⁻⁸ | 2,1.10⁻⁶ |
| 10 | 1,6.10⁻⁷ | 3,9.10⁻⁶ |
| 13 | 6,4.10⁻⁸ | 4,5.10⁻⁷ |
| 16 | 8,5.10⁻⁸ | 1,6.10⁻⁶ |
| 17 | 1,2.10⁻⁷ | 1,0.10⁻⁶ |
| 18 | 1,0.10⁻⁷ | 1,8.10⁻⁶ |
| 19 | 1,2.10⁻⁷ | 2,1.10⁻⁶ |
| 20 | 2,1.10⁻⁷ | 1,3.10⁻⁶ |
| 21 | 5,0.10⁻⁷ | 3,9.10⁻⁶ |
| 22 | 4,8.10⁻⁸ | 2,4.10⁻⁶ |
| 23 | 1,7.10⁻⁷ | 7,7.10⁻⁶ |
| 26 | 6,7.10⁻⁷ | 1,4.10⁻⁶ |
| 27 | 1,7.10⁻⁷ | 1,0.10⁻⁶ |
| 28 | 3,7.10⁻⁷ | 1,3.10⁻⁵ |
| 29 | 5,8.10⁻⁹ | 7,5.10⁻⁶ |

### EXEMPLE B : PROFIL DE LIAISON AUX RECEPTEURS CENTRAUX ADRENERGIQUES ALPHA 1 ET ALPHA 2. (ETUDE IN VITRO)

### OBJECTIF:

Mesurer, in vitro, l'affinité de liaison des composés de l'invention aux récepteurs centraux alpha 1 (α₁) et alpha 2 (α₂), en déterminant la capacité de ce produit à déplacer les radioligands spécifiques de ces récepteurs.

### RESULTATS :

Les résultats obtenus, in vitro sur les récepteurs adrénergiques avec nos conditions expérimentales, montrent que, les composés de l'invention n'ont qu'une très basse affinité pour les récepteurs alpha 1-adrénergiques (Kᵢ > 7 µM) et alpha 2-adrénergiques (Kᵢ > 10 µM).

### PROTOCOLE:

Le tableau suivant indique le radioligand utilisé pour marquer le récepteur, le produit et la concentration retenue pour déterminer la fraction non spécifique et le tissu choisi.

| Récepteur ou site | Radioligand | non spécifique | Structure |
|---|---|---|---|
| Alpha1 | [3H]-Prazosin | 10⁻⁵M Phentolamine | Cortex frontal veau |
| Alpha2 | [3H]-RX 821002 | 10⁻⁵M Yohimbine | Cortex frontal veau |

### EXEMPLE C : COMPOSITION PHARMACEUTIQUE : COMPRIMES

Comprimés dosés à 1 mg de 5-[(2-méthylphénoxy)méthyl]-4,5-dihydro-oxazol-2-ylamine

| | |
|---|---|
| 5-[(2-méthylphénoxy)méthyl]-4,5-dihydro-oxazol-2-ylamine | 1 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 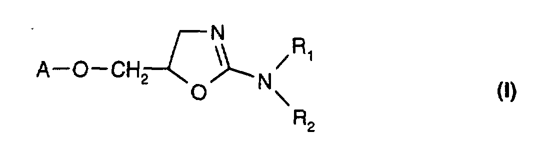 dans laquelle :
- R₁ et R₂ sont choisis indépendamment l'un de l'autre parmi l'hydrogène ou un radical alkyle,
- A est choisi parmi les radicaux : 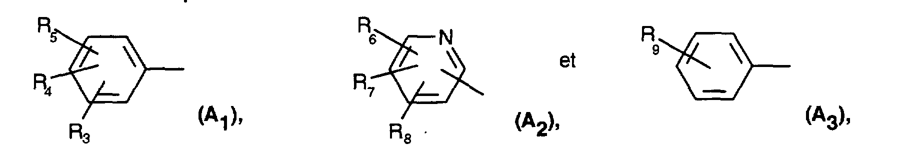 dans lesquels :
- R₃ représente un radical choisi parmi halogène, alkyle, alkoxy, amino, alkylamino, dialkylamino, carboxy, alkylcarbonyle, alkoxycarbonyle, trifluorométhyle, et cyano,
- R₄, R₅, R₆, R₇ et R₈ sont choisis chaucun indépendamment l'un de l'autre parmi l'hydrogène, un halogène, un radical alkyle, alkoxy, amino, alkylamino, dialkylamino, carboxy, alkoxycarbonyle, cyano et trifluorométhyle,
- R₉ représente le radical nitro,
avec la réserve que A ne peut pas représenter un groupement choisi parmi : dihalogénophényle, trihalogénophényle, 4-méthylphényle, 4-chlorophényle, 4-méthoxyphényle, 4-tert-butylphényle et 3-(diméthylamino)phényle,
leurs isomères optiques sous forme pure ou en mélange, ainsi que leurs sels d'addition à un acide ou une base, pharmaceutiquement acceptables,
étant entendu que :
les termes "alkyle" et "alkoxy" représentent des radicaux linéaires ou ramifiés contenant de 1 à 6 atomes de carbone.

2. Composés de formule (I) selon la revendication 1 dans laquelle A représente le radical de formule (A₄) : 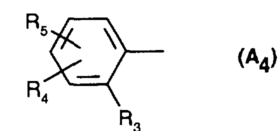 dans laquelle R₃, R₄ et R₅ sont tels que définis dans la formule (I).

3. Composés de formule (I) selon la revendication 1 dans laquelle A représente le radical de formule (A₆): 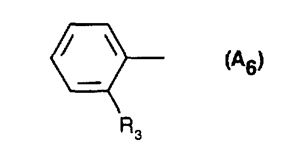 dans laquelle R₃ est tel que défini dans la revendication 1.

4. Composé selon la revendication 1 qui est la 5-[(2-méthylphénoxy)méthyl]-4,5-dihydrooxazol-2-ylamine.

5. Composé selon la revendication 1 qui est la (5*R**)-5-[(2-méthylphénoxy)méthyl]-4,5-dihydro-oxazol-2-ylamine.

6. Composé selon la revendication 1 qui est la 5-[(2,5-diméthylphénoxy)méthyl]-4,5-dihydro-oxazol-2-ylamine.

7. Composé selon la revendication 1 qui est la 5-[2,6-(diméthyl)phénoxyméthyl]-2-amino-2-oxazoline.

8. Composé selon la revendication 1 qui est la N-n-propyl-N-[5-(2-méthylphénoxy)méthyl-4,5-dihydro-oxazol-2-yl]amine.

9. Composé selon la revendication 1 qui est la N-éthyl-N-[5-(2-méthylphénoxy)méthyl-4,5-dihydro-oxazol-2-yl]amine.

10. Procédé de préparation des composés de formule (I) selon la revendication 1, **caractérisé en ce qu'**on fait réagir un composé de formule (II) : 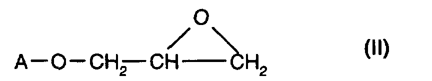 dans laquelle A est tel que défini dans la revendication 1 avec le cyanamide de sodium afin d'obtenir un composé de formule (I/a) : 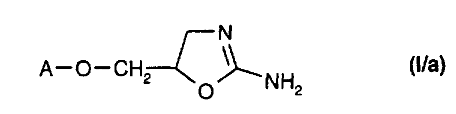 dans laquelle A est tel que défini précédemment, composé de formule (I/a) qui est ensuite mono- ou di-alkylé sur l'amine primaire afin d'obtenir le composé de formule (I/b) correspondant au composé de formule (I) dans lesquels R₁ et/ou R₂ représentent un alkyle, les composés de formule (I/a) et (I/b) formant l'ensemble des composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels isomères optiques, par des techniques classiques de séparation,
- ou transformés par un acide ou une base, en sels pharmaceutiquement acceptables.

11. Compositions pharmaceutiques contenant les produits de formule (I) selon l'une des revendications 1 à 9 ou le cas échéant un de leurs sels d'addition à un acide ou une base, pharmaceutiquement acceptables en combinaison avec un ou plusieurs excipients pharmaceutiquement acceptables.

12. Compositions pharmaceutiques selon la revendication 11 utiles pour le traitement des troubles liés aux récepteurs I₁.

13. Compositions pharmaceutiques selon l'une des revendications 11 ou 12 utiles dans les traitements de l'hypertension artérielle essentielle.

## Patentansprüche

1. Verbindungen der Formel (I): 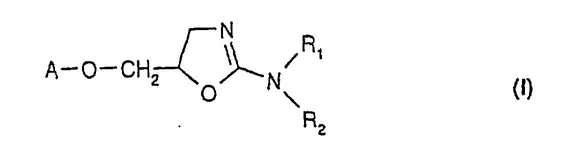 in der:
- R₁ und R₂ unabhängig voneinander aus Wasserstoff und Alkylgruppen ausgewählt sind,
- A ausgewählt ist aus den Gruppen: 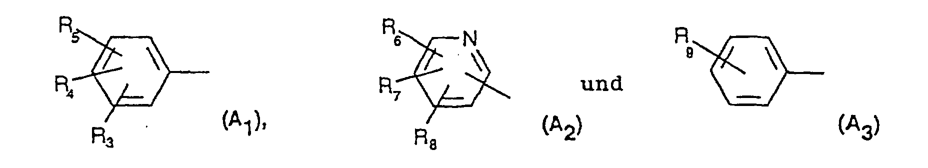
in denen:
- R₃ eine Gruppe ausgewählt aus Halogen, Alkyl, Alkoxy, Amino, Alkylamino, Dialkylamino, Carboxy, Alkylcarbonyl, Alkoxycarbonyl, Trifluormethyl und Cyano bedeutet,
- R₄, R₅, R₆, R₇ und R₈ unabhängig voneinander ausgewählt sind aus Wasserstoff, Halogen, Alkylgruppen, Alkoxygruppen, Aminogruppen, Alkylaminogruppen, Dialkylaminogruppen, Carboxygruppen, Alkoxycarbonylgruppen, Cyanogruppen und Trifluormethylgruppen, und
- R₉ eine Nitrogruppe bedeutet,
mit der Maßgabe, daß A keine Gruppe ausgewählt aus: Dihalogenphenyl, Trihalogenphenyl, 4-Methylphenyl, 4-Chlorphenyl, 4-Methoxyphenyl, 4-tert.-Butylphenyl und 3-(Dimethylamino)-phenyl bedeutet,
deren optische Isomere in reiner Form oder in Form einer Mischung sowie deren Additionssalze mit einer pharmazeutisch annehmbaren Säure oder Base,
wobei:
die Begriffe "Alkyl" und "Alkoxy" für geradkettige oder verzweigte Gruppen, die 1 bis 6 Kohlenstoffatome enthalten, stehen.

2. Verbindungen der Formel (I) nach Anspruch 1, worin A die Gruppe der Formel (A₄) darstellt: 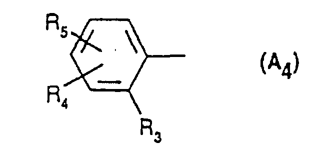 in der R₃, R₄ und R₅ die bezüglich der Formel (I) angegebenen Bedeutungen besitzen.

3. Verbindungen der Formel (I) nach Anspruch 1, worin A die Gruppe der Formel (A₆) darstellt: 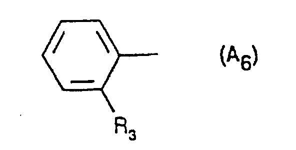 in der R₃ die in Anspruch 1 angegebenen Bedeutungen besitzt.

4. Verbindung nach Anspruch 1, nämlich 5-[(2-Methylphenoxy)-methyl]-4,5-dihydro-oxazol-2-ylamin.

5. Verbindung nach Anspruch 1, nämlich (5R*)-5-[(2-Methylphenoxy)-methyl]-4,5-dihydro-oxazol-2-ylamin.

6. Verbindung nach Anspruch 1, nämlich 5-[(2,5-Dimethylphenoxy)-methyl]-4,5-dihydro-oxazol-2-ylamin.

7. Verbindung nach Anspruch 1, nämlich 5-[2,6-(Dimethyl)-phenoxymethyl]-2-amino-2-oxazolin.

8. Verbindung nach Anspruch 1, nämlich N-n-Propyl-N-[5-(2-methylphenoxy)-methyl-4,5-dihydro-oxazol-2-yl]-amin.

9. Verbindung nach Anspruch 1, nämlich N-Ethyl-N-[5-(2-methylphenoxy)-methyl-4,5-dihydro-oxazol-2-yl]-amin.

10. Verfahren zur Herstellung der Verbindungen der Formel (I) nach Anspruch 1, **dadurch gekennzeichnet, daß** man eine Verbindung der Formel (II): 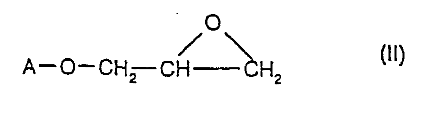 in der A die in Anspruch 1 angegebenen Bedeutungen besitzt, mit Natriumcyanamid umsetzt zur Bildung einer Verbindung der Formel (I/a): 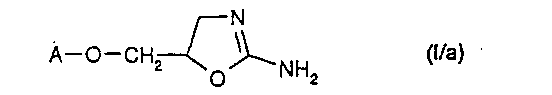 in der A die oben angegebenen Bedeutungen besitzt, welche Verbindung der Formel (I/a) anschließend am primären Amin mono- oder dialkyliert wird zur Bildung der Verbindung der Formel (I/b), welche der Verbindung der Formel (I) entspricht, in der R₁ und/oder R₂ Alkyl bedeuten, wobei die Verbindungen der Formeln (I/a) und (I/b) die Gesamtheit der Verbindungen der Formel (I) bilden,
welche Verbindungen der Formel (I) gewünschtenfalls
- mit Hilfe eines oder mehrerer Reinigungsverfahren ausgewählt aus Kristallisation, Chromatographie über Kieselgel, Extraktion, Filtration und Überführung über Aktivkohle oder ein Harz gereinigt werden können,
- gegebenenfalls mit Hilfe klassischer Trennmethoden in ihre eventuellen optischen Isomeren in reiner Form oder in Form einer Mischung getrennt werden können,
- oder mit einer Säure oder Base in pharmazeutisch annehmbare Salze umgewandelt werden können.

11. Pharmazeutische Zubereitungen enthaltend die Produkte der Formel (I) nach einem der Ansprüche 1 bis 9 oder gegebenenfalls eines ihrer pharmazeutisch annehmbaren Additionssalze mit einer Säure oder Base zusammen mit einem oder mehreren pharmazeutisch annehmbaren Trägermaterialien.

12. Pharmazeutische Zubereitungen nach Anspruch 11 zur Behandlung von mit den Rezeptoren I₁ verbundenen Störungen.

13. Pharmazeutische Zubereitungen nach Anspruch 11 oder 12 zur Behandlung der essentiellen arteriellen Hypertonie.

## Claims

1. Compounds of formula (I): 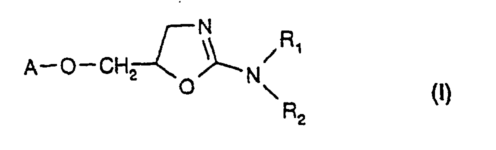 wherein :
- R₁ and R₂ are selected, each independently of the other, from hydrogen and an alkyl radical,
- A is selected from the radicals : 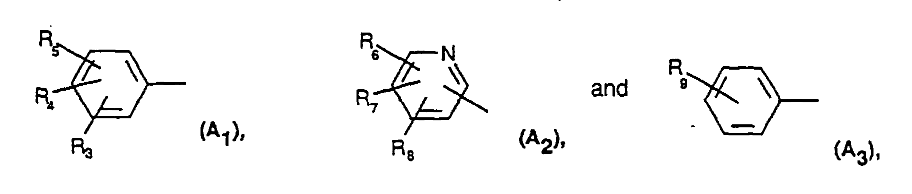
wherein:
- R₃ represents a radical selected from halogen, alkyl, alkoxy, amino, alkylamino, dialkylamino, carboxy, alkylcarbonyl, alkoxycarbonyl, trifluoromethyl and cyano,
- R₄, R₅, R₆, R₇ and R₈ are selected, each independently of the others, from hydrogen, a halogen, an alkyl, alkoxy, amino, alkylamino, dialkylamino, carboxy, alkoxycarbonyl, cyano and a trifluoromethyl radical,
- R₉ represents the nitro radical,
with the proviso that A cannot represent a grouping selected from : dihalophenyl, trihalophenyl, 4-methylphenyl, 4-chlorophenyl, 4-methoxyphenyl, 4-tert-butylphenyl and 3-(dimethylamino)phenyl,
optical isomers thereof in pure form or in the form of a mixture, and pharmaceutically acceptable addition salts thereof with an acid or base,
it being understood that:
the terms "alkyl" and "alkoxy" represent linear or branched radicals having from 1 to 6 carbon atoms.

2. Compounds of formula (I) according to claim 1, wherein A represents the radical of formula (A₄): 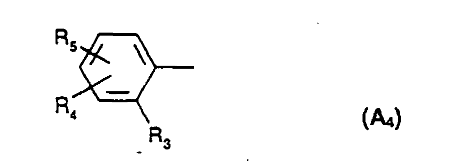 wherein R₃, R₄ and R₅ are as defined in formula (I).

3. Compounds of formula (I) according to claim 1, wherein A represents the radical of formula (A₆): 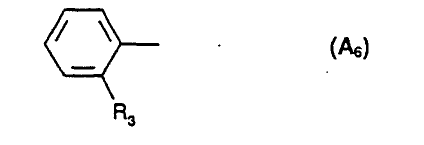 wherein R₃ is as defined in claim 1.

4. Compound according to claim 1 that is 5-[(2-methylphenoxy)methyl]-4,5-dihydro-oxazol-2-ylamine.

5. Compound according to claim 1 that is (5*R**)-5-[(2-methylphenoxy)methyl]-4,5-dihydro-oxazol-2-ylamine.

6. Compound according to claim 1 that is 5-[(2,5-dimethylphenoxy)methyl]-4,5-dihydro-oxazol-2-ylamine.

7. Compound according to claim 1 that is 5-[2,6-(dimethyl)phenoxymethyl]-2-amino-2-oxazoline.

8. Compound according to claim 1 that is N-n-propyl-N-[5-(2-methylphenoxy)-methyl-4,5-dihydro-oxazol-2-yl]amine.

9. Compound according to claim 1 that is N-ethyl-N-[5-(2-methylphenoxy)-methyl-4,5-dihydro-oxazol-2-yl]amine.

10. Process for the preparation of the compounds of formula (I) according to claim 1, **characterised in that** a compound of formula (II): 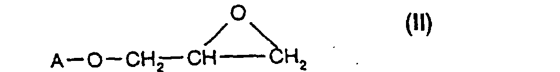 wherein A is as defined in claim 1 is reacted with sodium cyanamide to obtain a compound of formula (I/a): 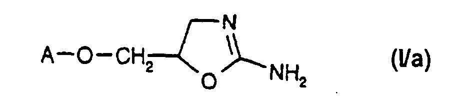 wherein A is as defined hereinbefore, which compound of formula (I/a) is then mono- or di-alkylated at the primary amine to obtain a compound of formula (I/b) corresponding to a compound of formula (I) wherein R₁ and/or R₂ represent alkyl, the compounds of formulae (I/a) and (I/b) constituting the totality of the compounds of formula (I),
which compounds of formula (I) may, if desired, be
- purified in accordance with one or more purification methods selected from crystallisation, chromatography over silica gel, extraction, filtration and passage over carbon or resin,
- separated, where appropriate, in pure form or in the form of a mixture, into their possible optical isomers, using conventional separation techniques, or
- converted into pharmaceutically acceptable salts with an acid or base.

11. Pharmaceutical compositions comprising compounds of formula (I) according to any one of claims 1 to 9 or, where appropriate, a pharmaceutically acceptable addition salt thereof with an acid or base, in combination with one or more pharmaceutically acceptable excipients.

12. Pharmaceutical compositions according to claim 11 for use in the treatment of disorders associated with I₁ -receptors.

13. Pharmaceutical compositions according to either of claims 11 and 12 for use in the treatment of essential arterial hypertension.
